# EUROPÄISCHE PATENTSCHRIFT

(11) **EP 2 795 307 B1**
(45) Veröffentlichungstag und Bekanntmachung des Hinweises auf die Patenterteilung: **18.10.2017**
(21) Anmeldenummer: 12798190.0
(22) Anmeldetag: 15.11.2012
(51) Int. Cl.: G01N 27/419, F01N 11/00, G01N 33/00, F02D 41/14, F02D 41/22

(54) **VERFAHREN ZUR ÜBERWACHUNG EINER BREITBANDLAMBDASONDE**
METHOD FOR MONITORING A BROADBAND LAMDBA PROBE
PROCÉDÉ DE SURVEILLANCE D'UNE SONDE LAMBDA LARGE BANDE

(30) Priorität: 21.12.2011 DE 102011089383
(43) Veröffentlichungstag der Anmeldung: 29.10.2014
(73) Patentinhaber: Robert Bosch GmbH, 70442 Stuttgart (DE)
(72) Erfinder: LEDERMANN, Bernhard, 70499 Stuttgart (DE); BEVOT, Claudius, 70197 Stuttgart (DE); REISCHL, Rolf, 70499 Stuttgart (DE)
(86) Internationale Anmeldenummer: PCT/EP2012/072671
(87) Internationale Veröffentlichungsnummer: WO 2013/092018

(56) Entgegenhaltungen:
- DE-A1- 19 800 027
- DE-A1-102010 028 301
- DE-T2- 69 727 588
- US-A1- 2010 073 017

## Beschreibung

### Stand der Technik

Die Erfindung betrifft ein Verfahren zur Bestimmung einer Polarisation einer Pumpzelle und/oder einer Nernstzelle einer Lambdasonde zur Diagnose der Breitband-Lambdasonde.

Die Ausgangssignale von Breitband-Lambdasonden werden durch eine externe Beschaltung ausgewertet, die zusätzlich dazu dient, die Betriebsparameter der Breitband-Lambdasonde einzustellen und zu überwachen sowie die Kabelverbindungen zu überwachen. Hierbei treten sowohl an der Nernstzelle als auch an der Pumpzelle der Breitband-Lambdasonde Polarisationsspannungen auf, die bei der Einstellung der Pumpspannung und der Auswertung der Ausgangssignale berücksichtigt werden müssen. Für die Polarisationsspannungen lassen sich zwar theoretische Werte für die unterschiedlichen Typen von Breitband-Lambdasonden festlegen, die realen Werte weichen von diesen jedoch durch Fertigungsstreuungen und Alterungseffekte an der Sonde zum Teil erheblich ab.

In der Schrift DE 10 2008 001697 A1 der Anmelderin wird eine verbesserte Beschaltung beschrieben, die es erlaubt, zusätzlich zu dem Betrieb des Abgassensors Informationen über den Betriebszustand der dort als Abgassensor verwendeten Breitbandlambdasonde zu erfassen, zu speichern und über eine Digitalschnittstelle an eine übergeordnete Motorsteuerung weiterzugeben. Diese Anordnung ermöglicht eine Diagnose der Kabelverbindungen zwischen der Beschaltung und der Breitbandlambdasonde auf Kurzschluss und Unterbrechung sowie auf Einhaltung der an den Anschlüssen zulässigen Spannungen. Die Betriebsbereitschaft der Abgassonde kann detektiert werden und deren Elektrodenpolarisation und die Alterung können kontinuierlich überwacht werden. Zur Durchführung dieser Messungen und zur Einstellung der verschiedenen Betriebszustände wird die Breitbandlambdasonde in aufeinander folgenden Schaltzuständen der Steuerelektronik unterschiedlich elektrisch beschaltet und entsprechend unterschiedlich elektrisch beaufschlagt. Dabei kann es zu einer Beeinflussung von Messungen durch vorherige Schaltzustände kommen. Beispielhaft kann ein Schaltzustand zu einer unerwünschten Polarisation einer Nernstzelle der Breitbandlambdasonde führen, die in einem nachfolgenden Schaltzustand zu einer Verfälschung des Messwerts der Nernstspannung an der Nernstzelle führt.
In der Schrift DE 102010000663A1 der Anmelderin wird eine Vorrichtung zum Betrieb einer Breitband-Lambdasonde im Abgaskanal einer Brennkraftmaschine und zur Erfassung von Informationen über den Betriebszustand der Breitband-Lambdasonde beschrieben. Die Vorrichtung ermöglichen eine Diagnose von Kabelverbindungen zwischen der Elektronik zur Steuerung und Auswertung der Signale der Breitband-Lambdasonde auf Unterbrechung und Kurzschluss. Weiterhin ermöglicht die Vorrichtung eine Anpassung einer Umladekorrektur wie sie aufgrund von Kapazitäten zwischen Zuleitungen und von Entstörmaßnahmen erforderlich sein kann.

Die Schrift DE 102010028301 A1 lehrt, dass die Alterung einer Breitbandlambdasonde zu einer geringeren Polarisationsspannung nach Beaufschlagung mit einem Stromimpuls führt.

Nach dem Stand der Technik ist eine Bestimmung der Polarisation einer Breitband-Lambdasonde im laufenden Betrieb bisher nur sehr eingeschränkt möglich. Bedingt durch Alterungseffekte an den Abgassensoren wäre eine solche Bestimmung zur Verbesserung der Messgenauigkeit bei unterschiedlichen Betriebspunkten der Brennkraftmaschine jedoch vorteilhaft.

Es ist Aufgabe der Erfindung, ein Verfahren bereitzustellen, welches eine Diagnose und Berücksichtigung einer Polarisationsspannung eines Sensorelements, insbesondere einer Pumpzelle und einer Nernstzelle in einer Breitband-Lambdasonde, im laufenden Betrieb ermöglicht.

### Offenbarung der Erfindung

Die Aufgabe der Erfindung wird dadurch gelöst, dass in einem ersten Verfahrensschritt die Pumpzelle und/oder die Nernstzelle mit einem Spannungs- oder Stromimpuls beaufschlagt wird und dass in einem zweiten Verfahrensschritt eine Spannung an der Pumpzelle und/oder der Nernstzelle bestimmt und als Maß für die Polarisation verwendet wird und dass die Funktion der Breitband-Lambdasonde über die bestimmte Polarisation überwacht wird. Die Bestimmung der Polarisation kann als einmalig oder mehrmalig ausgeführte Spannungsmessung oder durch die Bestimmung der Wirkung der Polarisation in einer zugehörigen Steuerung, wie einem Pumpstromregler einer Motorsteuerung, erfolgen. Die Spannungsmessung kann während einer Phase mit gleichzeitiger Beaufschlagung mit dem Strompuls oder in einer Pulspause erfolgen. Für Nernst- und Pumpzelle kann die Polarisation für Diagnosezwecke genutzt werden. Die Bestimmung der Polarisation der Pumpzelle kann über eine Ergänzung der Rechenvorschriften in dem zugeordneten Steuergerät zur Verbesserung der Genauigkeit des Lambdasignals verwendet werden.

In einer Weiterbildung des Verfahrens wird in dem ersten Verfahrensschritt die Pumpzelle und/oder die Nernstzelle mit einem Spannungs- oder Stromimpuls im regulären Betrieb der Breitband-Lambdasonde beaufschlagt oder es wird in dem ersten Verfahrensschritt der reguläre Betrieb der Breitband-Lambdasonde unterbrochen und in einem Diagnosezyklus die Pumpzelle und/oder die Nernstzelle mit einem gestellten Spannungs- oder Stromimpuls beaufschlagt. Die Diagnose der Breitband-Lambdasonde kann so auch während des laufenden Betriebs erfolgen, so dass eine Alterung festgestellt werden kann und eine Auswirkung der Polarisation bei der Bestimmung des Lambdawerts des Abgases berücksichtigt werden kann. Die Beaufschlagung mit dem Stromimpuls kann auch durch Vorgabe eines definierten, gestellten Pulses, wie er beispielhaft für eine Bestimmung einer Sprungantwort geeignet ist, erfolgen.

Wird in dem ersten Verfahrensschritt die Nernstzelle mit einem unidirektionalen Strompuls zum Stellen des Referenzpumpstroms oder zur Bestimmung des Innenwiderstands bestromt und wird in dem zweiten Verfahrensschritt zu vorgegebenen Zeiten nach dem unidirektionalen Strompuls die Spannung an der Nernstzelle oder eine mit der Polarisation in Zusammenhang stehende Größe bestimmt und als Maß für die Polarisation der Nernstzelle verwendet, kann während eines normalen Betriebs der Breitband-Lambdasonde die Polarisation der Nernstzelle bestimmt werden. Die Messung zur Charakterisierung der Polarisation erfolgt dabei zu vorbestimmten Zeiten relativ zum Strompuls vor und nach dem Strompuls. Die Bewertung der Polarisation in der zweiten Phase kann auch über den auf die Nernstspannung reagierenden Pumpstromregler unter Berücksichtigung von dessen Auslegung und des in ihm verwendeten Rechenalgorithmus erfolgen.

Eine Ausführungsform des Verfahrens zur Bestimmung der Polarisation der Pumpzelle sieht vor, dass in dem ersten Verfahrensschritt die Pumpzelle mit einem Pulspaar aus einem Stromimpuls und einem Gegenpuls beaufschlagt wird und dass in dem zweiten Verfahrensschritt die Spannung an der Pumpzelle zu vorgegebenen Zeiten vor und/oder nach dem Stromimpuls und dem Gegenpuls bestimmt wird und als Maß für die Polarisation der Pumpzelle verwendet wird. Hierbei kann die Höhe der Strompulse einstellbar sein.

In einer vorteilhaften Ausgestaltung des erfindungsgemäßen Verfahrens wird in dem ersten Verfahrensschritt die Pumpzelle mit einem Puls und einem Gegenpuls bestromt, in einem zweiten Verfahrensschritt wird in jeweils einer Pulspause zu einem vorgegebenen Zeitpunkt nach dem Puls eine erste Spannung über die Pumpzelle und zu einem vorgegebenen Zeitpunkt nach dem Gegenpuls eine zweite Spannung über die Pumpzelle gemessen und die Differenz zwischen der ersten Spannung und der zweiten Spannung wird als Maß für die Polarisation der Pumpzelle verwendet. In der praktischen Ausgestaltung wird bei einem stabilen Lambdawert in einem ersten Messzyklus, nach einem Stromimpuls, wie er im eingeregelten Betrieb verwendet wird, die Spannung Up01 an der unbestromten Pumpzelle bestimmt. In einem zweiten Messzyklus, nach einem vorgegebenen und damit bekannten Stromimpuls, wie er beispielhaft bei Fettgas verwendet wird, wird die Spannung Up02 an der unbestromten Pumpzelle bestimmt. Die Differenz der Spannungswerte Up01-Up02 ist ein Maß für die Polarisation der Breitband-Lambdasonde. Ist die Differenz klein, wird die Pumpzelle als schwach polarisiert eingestuft, ist die Differenz der Spannungswerte hoch, wird sie als stark polarisiert eingestuft.

Eine Ausführungsform des Verfahrens sieht vor, dass in dem zweiten Verfahrensschritt die Spannungswerte an der Pumpzelle zu mehreren vorgegebenen Zeiten während und nach dem jeweiligen Stromimpuls bestimmt werden und dass der Verlauf der Spannungswerte unter Berücksichtigung der Betriebsparameter der Breitband-Lambdasonde und/oder von Abgasparametern als Maß für die Polarisation der Pumpzelle verwendet wird. Hierdurch ist eine Charakterisierung einer Sprungantwort auf die Bestromung der Pumpzelle möglich, deren Spannungsverlauf die Information über die Polarisierbarkeit der Pumpzelle liefert. Zur Bewertung der Polarisierbarkeit wird zusätzlich die Temperatur der Lambdasonde sowie die Zusammensetzung des Abgases herangezogen.

Eine Erkennung von Abgassonden mit einem durch Alterung über das zulässige Maß angestiegenen Bedarf an Polarisierungsspannung ist erfindungsgemäß vorgesehen, indem die Polarisation der Breitband-Lambdasonde bestimmt und mit einem vorgegebenen Grenzwert verglichen wird und indem eine Breitband-Lambdasonde als fehlerhaft eingestuft wird, wenn die Polarisation über dem Grenzwert liegt.

Zur Dämpfung von Hochfrequenzstörungen und Hochspannungseinträgen sind an den Signal-Leitungen der Breitband-Lambdasonde Entstör-Kapazitäten vorgesehen. Wird der Pumpstrom einer Pumpzelle gepulst und nicht analog zeit- und wertekontinuierlich eingestellt, fließt durch die Pumpzelle zusätzlich ein Strom, der die Entstör-Kapazitäten und weitere massebezogene Kapazitäten umlädt. Dieser Umladestrom muss bei der Bestimmung des tatsächlichen Pumpstroms berücksichtigt werden. Die erforderliche Korrektur ist jedoch vom augenblicklichen Lambdawert des Abgases, dessen Temperatur und von der Polarisation der Pumpzelle abhängig. Insbesondere im Betriebspunkt Lambda = 1, bei dem idealerweise kein Pumpstrom fließt, wirken sich Fehlströme durch die Umladung der Kapazitäten besonders gravierend aus. Es ist daher vorteilhaft, wenn die Polarisation der Pumpzelle der Breitband-Lambdasonde bestimmt und wenn eine Umladekorrektur mit der Polarisation korrigiert wird.

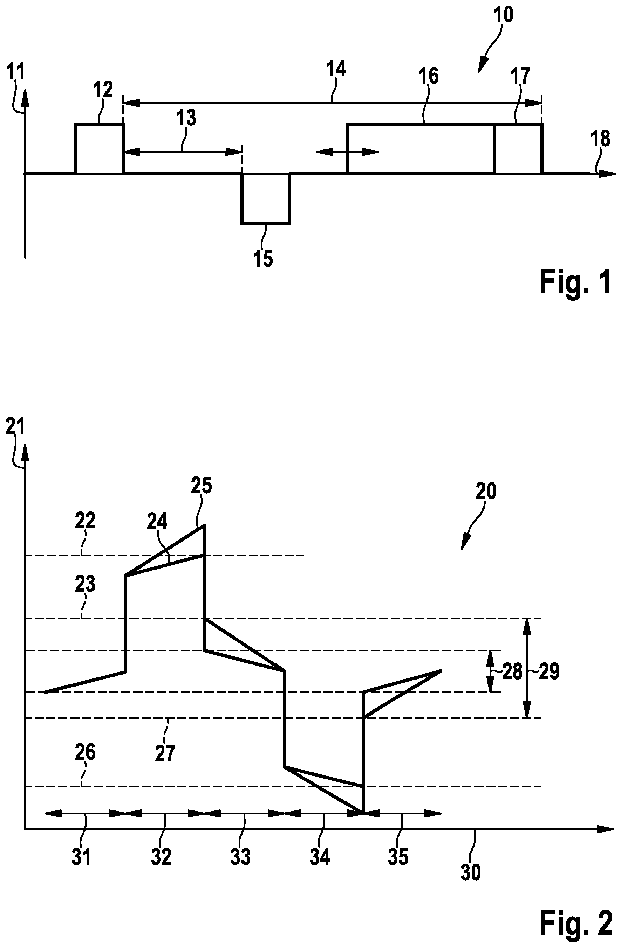

- Figur 2: ein Zeitdiagramm der Spannung an einer Pumpzelle einer Breitband-Lambdasonde.

Figur 1 zeigt in einem Pumpstromdiagramm 10 auf einer Stromachse 11 entlang einer ersten Zeitachse 18 einen zeitlichen Stromverlauf durch eine Pumpzelle einer Zweizellen-Breitband-Lambdasonde. Nach einem ersten Strompuls 12 beginnt eine Periode 14 des zeitlichen Stromverlaufs, die mit einem dritten Strompuls 17 endet. An den ersten Strompuls 12 schließt sich eine Pulspause 13 an, an die ein zweiter Strompuls 15 anschließt, der eine entgegengesetzte Polarität wie der dritte Strompuls 17 hat. Ein Pulsbeginn 16 des dritten Strompulses 17 ist zeitlich variabel einstellbar und bestimmt das Tastverhältnis während der Periode 14. Über den Pulsbeginn 16 wird der Gesamt-Pumpstrom durch die Pumpzelle während der Periode 14 gestellt oder unter Berücksichtigung der Nernstspannung einer Nernstzelle der Breitband-Lambdasonde geregelt. Der Gesamt-Pumpstrom kann zusätzlich durch die Höhe der Strompulse 12, 15 und 17 eingestellt werden. Die Strompulse 12, 15 und 17 stellen erfindungsgemäß die Anregung der Pumpzelle für die Bestimmung von deren Polarisation dar.

Figur 2 zeigt in einem Spannungsdiagramm 20 die Spannung an der Pumpzelle der Breitband-Lambdasonde auf einer Spannungsachse 21 entlang einer zweiten Zeitachse 30 während einer pulsförmigen Bestromung. In einer ersten Phase 31, einer dritten Phase 33 und einer fünften Phase 35 erfolgt die Spannungsmessung ohne Bestromung. In einer zweiten Phase 32 wird die Spannung während eines positiven Strompulses, in einer vierten Phase 34 während eines negativen Strompulses gemessen. An einer schwach polarisierten Pumpzelle ergibt sich ein erster Spannungsverlauf 24, an einer stark polarisierten Pumpzelle ein zweiter Spannungsverlauf 25. Charakteristisch für den ersten Spannungsverlauf 24 ist, dass eine erste Spannungsdifferenz 28 der Spannungen zu Beginn der unbestromten dritten Phase 33 und zu Beginn der ebenfalls unbestromten fünften Phase 35 aufgrund der jeweils davorliegenden bestromten zweiten Phase 32 und der vierten Phase 34 auftritt, die die Polarisierung der Pumpzelle kennzeichnet. Im Falle einer stärker polarisierten Pumpzelle mit dem zweiten Spannungsverlauf 25 ergibt sich eine zweite Spannungsdifferenz 29 zwischen den Spannungen zu Beginn der unbestromten dritten Phase 33 und zu Beginn der ebenfalls unbestromten fünften Phase 35, wobei die zweite Spannungsdifferenz 29 größer als die ersten Spannungsdifferenz 28 ist.

Die Bewertung der Spannungsdifferenzen 28, 29 kann, bei Vergleich mit einem vorgegebenen Grenzwert, dazu dienen, die Alterung von Breitband-Lambdasonden zu bewerten und gegebenenfalls eine Sonde als fehlerhaft einzustufen. Zum Ende der bestromten zweiten und vierten Phasen 32, 34 stellen sich eine Spannung nach dem ersten Strompuls 22 und eine Spannung nach dem zweiten Strompuls 26 ein, deren Höhe von der Polarisierung der Pumpzelle abhängen. Die Bewertung des gesamten Spannungsverlaufs 24, 25 über bestromte und unbestromte Phasen 31, 32, 33, 34 und 35 kann ebenfalls zu einer Bewertung der Polarisation herangezogen werden.

Bei einer pulsförmigen Bestromung müssen massebezogene Kapazitäten an der Breitband-Lambdasonde, wie sie beispielhaft zur Dämpfung von Hochfrequenzstörungen und Hochspannungseinträgen an den Signal-Leitungen der Breitband-Lambdasonde als Entstör-Kapazitäten vorgesehen sind, berücksichtigt werden. Die hierdurch auftretenden so genannten Umladefehler müssen in einer Umladekorrektur zur Bestimmung des korrekten mittleren Pumpstroms berücksichtigt werden. Der Umladefehler ist vom Betriebszustand der Brennkraftmaschine über die Abgaszusammensetzung, von der Temperatur des Abgases und der Breitband-Lambdasonde sowie von der Polarisationsspannung an der Pumpzelle abhängig. Durch die erfindungsgemäß verbesserte Bestimmung der Polarisation der Pumpzelle kann somit auch die Umladekorrektur verbessert werden.

## Patentansprüche

1. Verfahren zur Bestimmung einer Polarisation einer Pumpzelle und/oder einer Nernstzelle einer Breitband-Lambdasonde zur Diagnose der Breitband-Lambdasonde, wobei in einem ersten Verfahrensschritt die Pumpzelle und/oder die Nernstzelle mit einem Spannungs- oder Stromimpuls beaufschlagt wird und wobei in einem zweiten Verfahrensschritt die Spannung oder ein Spannungsverlauf an der Pumpzelle und/oder der Nernstzelle bestimmt und als Maß für die Polarisation verwendet wird, **dadurch gekennzeichnet, dass** die Funktion der Breitband-Lambdasonde über die derart bestimmte Polarisation überwacht wird, indem die bestimmte Polarisation der Breitband-Lambdasonde mit einem vorgegebenen Grenzwert verglichen wird und eine Breitband-Lambdasonde als fehlerhaft eingestuft wird, wenn die Polarisation über dem Grenzwert liegt.

2. Verfahren nach Anspruch 1, **dadurch gekennzeichnet, dass** in dem ersten Verfahrensschritt die Pumpzelle und/oder die Nernstzelle mit einem Spannungs- oder Stromimpuls im regulären Betrieb der Breitband-Lambdasonde beaufschlagt wird oder dass in dem ersten Verfahrensschritt der reguläre Betrieb der Breitband-Lambdasonde unterbrochen wird und in einem Diagnosezyklus die Pumpzelle und/oder die Nernstzelle mit einem gestellten Spannungs- oder Stromimpuls beaufschlagt wird.

3. Verfahren nach Anspruch 1 oder 2, **dadurch gekennzeichnet, dass** in dem ersten Verfahrensschritt die Nernstzelle mit einem unidirektionalen Strompuls zum Stellen des Referenzpumpstroms oder zur Bestimmung des Innenwiderstands bestromt wird und dass in dem zweiten Verfahrensschritt zu vorgegebenen Zeiten nach dem unidirektionalen Strompuls die Spannung an der Nernstzelle bestimmt wird und als Maß für die Polarisation der Nernstzelle verwendet wird.

4. Verfahren nach einem der Ansprüche 1 bis 3, **dadurch gekennzeichnet, dass** in dem ersten Verfahrensschritt die Pumpzelle mit einem Pulspaar aus einem Stromimpuls und einem Gegenpuls beaufschlagt wird und dass in dem zweiten Verfahrensschritt die Spannung an der Pumpzelle zu vorgegebenen Zeiten vor und/oder nach dem Stromimpuls und dem Gegenpuls bestimmt wird und als Maß für die Polarisation der Pumpzelle verwendet wird.

5. Verfahren nach einem der Ansprüche 1 bis 4, **dadurch gekennzeichnet, dass** in dem ersten Verfahrensschritt die Pumpzelle mit einem Puls und einem Gegenpuls bestromt wird, dass in einem zweiten Verfahrensschritt in jeweils einer Pulspause zu einem vorgegebenen Zeitpunkt nach dem Puls eine erste Spannung über die Pumpzelle und zu einem vorgegebenen Zeitpunkt nach dem Gegenpuls eine zweite Spannung über die Pumpzelle gemessen wird und dass die Differenz zwischen der ersten Spannung und der zweiten Spannung als Maß für die Polarisation der Pumpzelle verwendet wird.

6. Verfahren nach einem der Ansprüche 1 bis 5, **dadurch gekennzeichnet, dass** in dem zweiten Verfahrensschritt die Spannungswerte an der Pumpzelle zu mehreren vorgegebenen Zeiten während und nach dem jeweiligen Stromimpuls bestimmt werden und dass der Verlauf der Spannungswerte unter Berücksichtigung der Betriebsparameter der Breitband-Lambdasonde und/oder von Abgasparametern als Maß für die Polarisation der Pumpzelle verwendet wird.

7. Verfahren nach einem der Ansprüche 1 bis 6, **dadurch gekennzeichnet, dass** die Polarisation der Pumpzelle der Breitband-Lambdasonde bestimmt und dass eine Umladekorrektur mit der Polarisation korrigiert wird.

## Claims

1. Method for determining a polarization of a pump cell and/or of a Nernst cell of a broadband lambda probe for the purposes of diagnosis of the broadband lambda probe, wherein, in a first method step, the pump cell and/or the Nernst cell have/has a voltage or current pulse applied thereto, and wherein, in a second method step, the voltage or a voltage profile at the pump cell and/or the Nernst cell is determined and used as a measure for the polarization, **characterized in that** the function of the broadband lambda probe is monitored on the basis of the polarization thus determined, by virtue of the determined polarization of the broadband lambda probe being compared with a predefined threshold value and a broadband lambda probe being classed as being faulty if the polarization overshoots the threshold value.

2. Method according to Claim 1, **characterized in that**, in the first method step, the pump cell and/or the Nernst cell has a voltage or current pulse applied thereto during normal operation of the broadband lambda probe, or **in that**, in the first method step, the normal operation of the broadband lambda probe is interrupted and, in a diagnosis cycle, the pump cell and/or the Nernst cell have/has a set voltage or current pulse applied thereto.

3. Method according to Claim 1 or 2, **characterized in that**, in the first method step, the Nernst cell is energized with a unidirectional current pulse for the purposes of setting the reference pump current or for the purposes of determining the internal resistance, and **in that**, in the second method step, at predefined times after the unidirectional current pulse, the voltage at the Nernst cell is determined and is used as a measure for the polarization of the Nernst cell.

4. Method according to one of Claims 1 to 3, **characterized in that**, in the first method step, the pump cell has a pulse pair composed of a current pulse and a counter-pulse applied thereto, and **in that**, in the second method step, the voltage at the pump cell is determined at predefined times before and/or after the current pulse and the counter-pulse and is used as a measure for the polarization of the pump cell.

5. Method according to one of Claims 1 to 4, **characterized in that**, in the first method step, the pump cell is energized with a pulse and a counter-pulse, **in that**, in a second method step, in a respective pulse interval, a first voltage across the pump cell is measured at a predefined point in time after the pulse and a second voltage across the pump cell is measured at a predefined point in time after the counter-pulse, and **in that** the difference between the first voltage and the second voltage is used as a measure for the polarization of the pump cell.

6. Method according to one of Claims 1 to 5, **characterized in that**, in the second method step, the voltage values at the pump cell are determined at multiple predefined times during and after the respective current pulse, and **in that** the profile of the voltage values is used, taking into consideration the operating parameters of the broadband lambda probe and/or exhaust gas parameters, as a measure for the polarization of the pump cell.

7. Method according to one of Claims 1 to 6, **characterized in that** the polarization of the pump cell of the broadband lambda probe is determined, and **in that** a charge reversal correction is corrected by means of the polarization.

## Revendications

1. Procédé de détermination de la polarisation d'une cellule de pompage et/ou d'une cellule de Nernst d'une sonde Lambda à large bande en vue de diagnostiquer la sonde Lambda à large bande, dans une première étape du procédé, la cellule de pompage et/ou la cellule de Nernst étant alimentées avec une première impulsion de tension ou de courant et, dans une deuxième étape du procédé, la tension ou une courbe de tension étant déterminée au niveau de la cellule de pompage et/ou de la cellule de Nernst et utilisée comme mesure pour la polarisation, **caractérisé en ce que** le fonctionnement de la sonde Lambda à large bande est surveillé par le biais de la polarisation ainsi déterminée **en ce que** la polarisation déterminée de la sonde Lambda à large bande est comparée à une valeur limite prédéfinie et une sonde Lambda à large bande est classifiée défaillante lorsque la polarisation est supérieure à la valeur limite.

2. Procédé selon la revendication 1, **caractérisé en ce que** dans la première étape de procédé, la cellule de pompage et/ou la cellule de Nernst sont alimentées par une impulsion de tension ou de courant durant le fonctionnement normal de la sonde Lambda à large bande, ou **en ce que** dans la première étape de procédé, le fonctionnement normal de la sonde Lambda à large bande est interrompu et la cellule de pompage et/ou la cellule de Nernst sont alimentées par une impulsion de tension ou de courant réglée dans un cycle de diagnostic.

3. Procédé selon la revendication 1 ou 2, **caractérisé en ce que** dans la première étape de procédé, la cellule de Nernst est alimentée avec une impulsion de courant unidirectionnelle en vue de régler le courant de pompage de référence ou pour déterminer la résistance interne, et **en ce que** dans la deuxième étape de procédé, la tension au niveau de la cellule de Nernst est déterminée à des instants prédéfinis après l'impulsion de courant unidirectionnelle et utilisée comme mesure pour la polarisation de la cellule de Nernst.

4. Procédé selon l'une des revendications 1 à 3, **caractérisé en ce que** dans la première étape de procédé, la cellule de pompage est alimentée avec une paire d'impulsions constituée d'une impulsion de courant et d'une contre-impulsion, et **en ce que** dans la deuxième étape de procédé, la tension au niveau de la cellule de pompage est déterminée à des instants prédéfinis avant et/ou après l'impulsion de courant et la contre-impulsion et utilisée comme mesure pour la polarisation de la cellule de pompage.

5. Procédé selon l'une des revendications 1 à 4, **caractérisé en ce que** dans la première étape de procédé, la cellule de pompage est alimentée avec une impulsion et une contre-impulsion, et **en ce que** dans une deuxième étape de procédé, respectivement dans une pause d'impulsion, une première tension aux bornes de la cellule de pompage est mesurée à un instant prédéfini après l'impulsion et une deuxième tension aux bornes de la cellule de pompage à un instant prédéfini après la contre-impulsion, et **en ce que** la différence entre la première tension et la deuxième tension est utilisée comme mesure pour la polarisation de la cellule de pompage.

6. Procédé selon l'une des revendications 1 à 5, **caractérisé en ce que** dans la deuxième étape de procédé, les valeurs de tension au niveau de la cellule de pompage sont déterminées à plusieurs instants prédéfinis pendant et après l'impulsion de courant respective et **en ce que** la courbe des valeurs de tension est utilisée comme mesure pour la polarisation de la cellule de pompage en tenant compte des paramètres de fonctionnement de la sonde Lambda à large bande et/ou de paramètres des gaz d'échappement.

7. Procédé selon l'une des revendications 1 à 6, **caractérisé en ce que** la polarisation de la cellule de pompage de la sonde Lambda à large bande est déterminée et **en ce qu'**une correction d'inversion de charge est corrigée avec la polarisation.
